# EUROPEAN PATENT SPECIFICATION

(11) **EP 2 978 407 B1**
(45) Date of publication and mention of the grant of the patent: **06.05.2020**
(21) Application number: 14737344.3
(22) Date of filing: 27.03.2014
(51) Int. Cl.: A61K 9/08, A61K 9/00, A61K 47/12, A61K 47/10, A61K 47/18, A61K 31/46, A61K 31/4174

(54) **AQUEOUS SOLUTION COMPOSITION CONTAINING IPRATROPIUM AND OXYMETAZOLINE**
WÄSSRIGE LÖSUNGSZUSAMMENSETZUNG MIT IPRATROPIUM UND OXYMETAZOLIN
COMPOSITION DE SOLUTION AQUEUSE CONTENANT DE L'IPRATROPIUM ET DE L'OXYMÉTAZOLINE

(30) Priority: 29.03.2013 TR 201303840
(43) Date of publication of application: 03.02.2016
(73) Proprietor: Santa Farma Ilaç Sanayi A.S., 34382 Istanbul (TR)
(72) Inventor: BUKET, Aksu, Sisli 34382 Istanbul (TR); LALE, Karaoguz, Sisli 34382 Istanbul (TR); BANU, Sunman, Öztuna, Sisli 34382 Istanbul (TR); AYSE, Yilmaz, Sisli 34382 Istanbul (TR); GÜHER, Isik, Cesur, Sisli 34382 Istanbul (TR)
(74) Representative: Bulut, Pinar
(86) International application number: PCT/TR2014/000111
(87) International publication number: WO 2014/158119

(56) References cited:
- WO-A1-99/38492
- US-A1- 2004 053 902
- US-A1- 2004 248 924

## Description

### Field of invention

The invention relates to an aqueous solution composition as defined in the claims for the symptomatic treatment of common cold symptoms through mucosal delivery, preferably suitable for local administration into the nose.

### Background of the invention

Ipratropium, a derivative of N-isopropyl noratropine, has an anticholinergic effect on upper respiratory tract. The basic patent of Ipratropium is US 3505337. For the symptomatic treatment of rhinorrhea associated with perennial rhinitis, a nasal spray (Atrovent®) containing Ipratropium bromide (Formule I) is available on the market.
Oxymetazoline (Formula II) is a vasoconstrictor imidazole-Sympathomimetics, which is preferably used locally in the nose for the decongestion of the mucosa. The basic patent of Oxymetazoline is DE 1117588. Nasal preparations with decongestant activity containing Oxymetazoline hydrochloride (Iliadin Merck® Metered-dose Nasal Spray, Drops, Pediatric Spray) are available on the market.

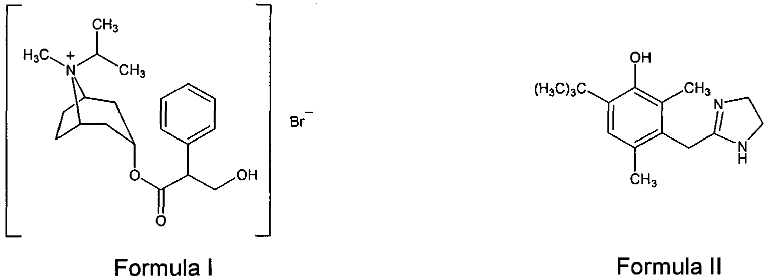

A nasal spray containing Ipratropium in combination with Xylometazoline, a Oxymetazoline similar molecule available on the market (Otrivin Comp®).

The document US 2004/0248924 A1 discloses in the table 9 a composition comprising ipratropium bromide and oxymetazoline.

Co-administration of Ipratropium and Xylometazoline has been investigated by Pitkäranta et al. (Pitkäranta et al.., Am J Rhinol., Mar-Apr;12(2): 125-9, 1998). However, a preparation comprising a combination of Oxymetazoline or a salt thereof with Ipratropium or a salt thereof used for the symptomatic treatment of common cold and/or of rhinitis and in cases related to these symptoms such as nasal congestion, sneezing and hypersecretion (rhinorrhea) is not present currently on the market.

### Detailed description of the invention

This invention relates to dosage forms used for the treatment of common cold symptoms via mucosal delivery. The composition according to the invention is defined in the claims.

The term "Ipratropium or a salt thereof' is intended to relate to Ipratropium, a pharmaceutically acceptable salt thereof, a mixture of Ipratropium and one or more pharmaceutically acceptable salts thereof, or a mixture of pharmaceutically acceptable salts of Ipratropium. Pharmaceutically acceptable salt of Ipratropium are Ipratropium bromide, Ipratropium chloride, Ipratropium iodide, Ipratropium fluoride, or Ipratropium hydroxide. Likewise, the term "Oxymetazoline or a salt thereof" is intended to relate to Oxymetazoline, a pharmaceutically acceptable salt thereof, a mixture of Oxymetazoline and one or more salts thereof, or a mixture pharmaceutically acceptable salts of Oxymetazoline. Pharmaceutically acceptable salt of Oxymetazoline are Oxymetazoline hydrochloride, Oxymetazoline hydrobromide, Oxymetazoline hydroiodide, Oxymetazoline hydrofluoride, Oxymetazoline sulphate, Oxymetazoline nitrate, Oxymetazoline formate, Oxymetazoline acetate, Oxymetazoline citrate, Oxymetazoline tartrate, or Oxymetazoline fumarate.

The term "pharmaceutically acceptable salts" is denoted to mean substances that are essentially non-toxic following administration to a mucosa and meet a specified chemical or microbial quality. The term "complex binders" is denoted to mean substances that are capable of binding inorganic metal ions i. e. alkali metal ions, earth alkali metal ions or heavy metal ions. The complex binder is edetic acid, pentetic acid, nitrilotriacetic acid, malonic acid, succinic acid, glutaric acid, adipic acid, pimelic acid, tartaric acid, malic acid, citric acid and/or their salts.

The term "humectant" relates to an agent that brings about a moisturising effect to the target where it is applied. Typically humectants are sorbitol, propylene glycol, glycerol, glycerine, polyethylene glycols, triacetin, hydroxypropylmethylcellulose methylcellulose, polyvinyl alcohol, polyvinylpyrrolidone, poloxamer. According to the invention, the humectant is glycerol.

The term " preservative" relates to any kind of agent in a composition or in a dosage form that can prevent or reduce the physical and/or chemical degradation of the active substances. Typically preservatives are benzalkonium chloride, benzyl alcohol, potassium sorbate, sorbic acid, chlorobutanol, phenoxyethanol, phenylethyl alcohol, chlorocresol, boric acid, phenyl mercuric borate, phenyl mercuric acetate, phenyl mercuric nitrate, methyl paraben, ethyl paraben, propyl paraben, butyl paraben and their salts. According to the invention, the preservative is benzalkonium chloride.

The term "pH-adjusting agent" relates to any suitable inorganic base, inorganic acid, organic base or organic acid, including acids and bases with one or multiple pKa values. The pH of the solution is adjusted between 4.0 and 7.5 by using a suitable pH-adjusting agent like phosphate buffer, citrate buffer. Suitable buffering agents that can be used are sodium dihydrogen phosphate, disodium hydrogen phosphate, citric acid, sodium citrate, sodium hydroxide, hydrochloric acid or a mixture thereof.

The term "formulated" is intended to relate to the selection of excipients, carriers, vehicles, solvents, co-solvents, preservatives, colouring agents, flavouring agents and so forth in the preparation of a medicament using said composition.

The term "dosage" relates to the quantity of active drugs administered to a mucosa by means of one delivery operation. In the embodiment, wherein the active drugs are formulated for administration to a nasal mucosa, the term "dosage" relates to the quantity of active drugs administered to one nostril by means of one delivery operation.

The term "dosage unit" relates to a composition administered by means of one or more delivery operation. In the embodiment, wherein the composition is a liquid, "a dosage unit" is the volume of the composition administered by means of one or more delivery operation.

The term "delivery operation" is an operation, which delivers a dosage to a mucosa.

The term "mucosal delivery" relates to delivery of a composition to a mucous membrane, such as the buccal or labial mucosa or the mucosa of the respiratory tract, such as the nasal mucosa. In the context of this invention, the use of a complex binder together with other excipients (e.g. preservative, pH-adjusting agent, etc.) in order to stabilise an aqueous solution comprising Ipratropium bromide and Oxymetazoline hydrochloride.

Aqueous solution comprises as active ingredient, 0.05 mg/ml to 5 mg/ml Ipratropium bromide and 0.05 mg/ml to 5 mg/ml Oxymetazoline hydrochloride. pH of the solution is between 4.0 to 7.5.

An aqueous solution composition according to the invention is used for the treatment of the symptoms related with common cold and/or of rhinitis. It is applied in cases related to these symptoms such as nasal congestion, sneezing and hypersecretion (rhinorrhea).

The example given below, illustrates the invention without being limited to the compounds and their amounts mentioned.

**Example:**

| Ingredients | Amount, % w/v (mg/ml) |
|---|---|
| Ipratropium bromide | 0.005% - 0.5% w/v (0.05 mg/ml - 5 mg/ml) |
| Oxymetazoline hydrochloride | 0.005% - 0.5% w/v (0.05 mg/ml - 5 mg/ml) |
| Disodium edetate | 0.005% - 0.1% w/v |
| Glycerol | 0% - 30.0% w/v |
| Benzalkonium chloride | 0%- 0.02% w/v |
| Buffer (for pH adjustment) | q.s. |
| Water | q.s. |

## Claims

1. An aqueous solution composition formulated for mucosal delivery comprising:
**a)** 0.05 mg/ml to 5 mg/ml Ipratropium bromide,
**b)** 0.05 mg/ml to 5 mg/ml Oxymetazoline hydrochloride, and
**c)** Disodium edetate as complex binder, glycerol as humectant, benzalkonium chloride as preservative and a pH adjusting agent,
said aqueous solution has a pH range from 4.0 to 7.5.

2. The composition according to claim 1, wherein the pH-adjusting agent is selected from the group consisting of sodium dihydrogen phosphate, disodium hydrogen phosphate, citric acid, sodium citrate, sodium hydroxide, hydrochloric acid or a mixture thereof.

3. The composition according to claims 1-2, wherein it comprises:
**a)** Ipratropium bromide,
**b)** Oxymetazoline hydrochloride,
**c)** Disodium edetate as complex binder, glycerol as humectant, benzalkonium chloride as preservative and pH 4.0 to 7.5 phosphate buffer.

4. The composition according to to claims 1-2, wherein it comprises:
**a)** Ipratropium bromide,
**b)** Oxymetazoline hydrochloride,
**c)** Disodium edetate as complex binder, glycerol as humectant, benzalkonium chloride as preservative and pH 4.0 to 7.5 citrate buffer.

5. The composition according to any one of the preceding claims, wherein it is applied to nasal mucosa.

6. The composition according to any one of the preceding claims for use in the treatment of conditions selected from the group consisting of symptoms associated with the common cold and symptoms associated with rhinitis in human.

7. The composition for use according to claim 6, wherein the conditions are nasal congestion, sneezing and hypersecretion (rhinorrea).

## Patentansprüche

1. Für die mukosale Anwendung formulierte Zusammensetzung einer wässrigen Lösung beinhaltet folgendes:
a. 0.05 mg/ml bis 5 mg/ml Ipratropiumbromid,
b. 0.05 mg/ml bis 5 mg/ml Oximetazolinhydrochlorid und
c. als komplexes Bindemittel Dinatrium-EDTA, als feuchtmittel Glyzerol, als Konservierungsmittel und als ein pH-Einstellungsagent Benzalkoniumchlorid;
der pH-Wert der oben genannten wässrigen Lösung liegt im Bereich von 4.0 bis 7.5.

2. Der pH-Einstellunngsagent der zusammensetzung nach anspruch 1 wird aus der Gruppe von Natriumdihydrogenphosphat, Dinatriumhydrogenphosphat, Zitronensäure, Natriumcitrat, Natriumhydroxid - Salzsäure oder von deren Mischung gewählt.

3. Zusammensetzung nach anspruch 1-2 beinhaltet folgendes:
**a.** Ipratropium bromid,
**b.** Oximetazolin hydrochlorid,
**c.** als komplexes Bindemittel Dinatrium-EDTA, als feuchtmittel Glyzerol, als Konservierungsmittel Benzalkoniumchlorid und Phosphat-Puffer in einem pH-Bereich von 4.0 bis 7.5.

4. Zusammensetzung nach anspruch 1-2 beinhaltet folgendes:
**a.** Ipratropiumbromid,
**b.** Oximetazolinhydrochlorid,
**c.** als komplexes Bindemittel Dinatrium-EDTA, als feuchtmittel Glycerol, als Konservierungsmittel Benzalkoniumchlorid und Citrat-Puffer in einem pH-Bereich von 4.0 bis 7.5.

5. Die Zusammensetzung nach einem der oben angegebenen ansprüche wird in die Nasenschleimhaut angewendet.

6. Die Zusammensetzung nach einem der oben angegebenen ansprüche wird zur Behandlung der Symptome im Zusammenhang mit der Erkältung und der Krankheiten angewendet, die aus der Gruppe bestehend aus der Symptome im Zusammenhang mit der der Rhinitis beim Menschen gewählt werden.

7. Die Zusammensetzung nach anspruch 6 wird zur Behandlung der Nasenverstopfung, des Niesens und der Hypersekretion (Rhinorrhoe) angewendet.

## Revendications

1. Composition de solution aqueuse formulée pour l'administration muqueuse, comprenant:
a. 0,05 mg / ml à 5 mg / ml de bromure d'ipratropium,
b. 0,05 mg / ml à 5 mg / ml, chlorhydrate d'oxymétazoline, et
c. l'édétate disodique comme liant complexe, le glycérol comme humectant, le chlorure de benzalkonium comme conservateur et un agent d'ajustement du pH, ladite solution aqueuse a une gamme de pH de 4.0 à 7.5.

2. Composition selon la demande 1, dans laquelle l'agent d'ajustement du pH est choisi dans le groupe constitué par le dihydrogénophosphate de sodium, l'hydrogénophosphate disodique, l'acide citrique, le citrate de sodium, l'hydroxyde de sodium, l'acide chlorhydrique ou un mélange de ceux-ci.

3. Composition selon les demandes 1-2, dans laquelle elle comprend:
a. Bromure d'ipratropium
b. Chlorhydrate d'oxymétazoline
c. Edétate disodique comme liant complexe, glycérol comme humectant, chlorure de benzalkonium comme conservateur et tampon phosphate de pH 4.0 à 7.5.

4. Composition selon les demandes 1-2, dans laquelle elle comprend:
a. Bromure d'ipratropium
b. Chlorhydrate d'oxymétazoline
c. Edétate disodique comme liant complexe, glycérol comme humectant, chlorure de benzalkonium comme conservateur et tampon citrate de pH 4.0 à 7.5.

5. Composition selon l'une ou l'autre des demandes précédentes, dans laquelle elle est appliquée sur la muqueuse nasale.

6. Composition selon l'une ou l'autre des demandes précédentes, destinée à être utilisée dans le traitement d'affections choisies dans le groupe constitué des symptômes associés au rhume et des symptômes associés à la rhinite chez l'homme.

7. Composition à utiliser selon la demande 6, dans laquelle les conditions sont la congestion nasale, les éternuements et l'hypersécrétion (rhinorrhée).
